# EUROPEAN PATENT APPLICATION

(11) **EP 2 224 364 A1**
(43) Date of publication of application: **01.09.2010**
(21) Application number: 09171141.6
(22) Date of filing: 23.09.2009
(51) Int. Cl.: G06F 19/00

(54) **Method for the on-line drafting of a medical report**

(30) Priority: 23.09.2008 IT PI20080096
(71) Applicant: Acomep S.r.l., 56122 Ghezzano (PI) (IT)
(72) Inventor: Cannavo', Giovanni, 56122 Pisa (IT); Velucchi, Mario, 56121 Pisa (IT); Mastroroberto, Luigi, 40126 Bologna (IT)
(74) Representative: Celestino, Marco

(57) **Abstract**

A method for on-line drafting a medical report comprising a first step in which a user, for example a medical examiner who is in charge of the examination, accesses through a web interface to a visualization file (5) that is run in a server. In particular, the file has a structure that is suitable for allowing the visualization in one data sheet, which assists the user to fill in fields that are intended to receive data (1001-1030) and to obtain a small size filled in visualization file. Moreover, this assists the elaboration of said file by means of an interface database that provides to update the filled in fields and to test formal correctness of the filling in process. Afterwards, the filled in visualization file (5) is transmitted in an encrypted format to the server where it may be displayed by insurance companies [Fig. 1].

## Description

### Field of the invention

The present invention relates to a method for on-line drafting a medical report.

### Description of the prior art

The need is felt of an evaluation method by use in medical examinations such as those concerning automobile liability insurances or accident insurances.

As known in the field of damages to the public in automobile liability insurances, most of activity relates to disputes on "small damages", "small disablements" or on the so called "micro-permanent" impairments. In particular, about 80% of medical examinations for insurance companies relate to cases where permanent biological damages are recognized within the value of 3%.

Medical examiners are trying to give a real significance to such values wondering whether or not they relate to actual pathological phenomena that cause the health of the damaged persons to a permanent worsening. Only in the affirmative, in fact, the damages should be recognised as if the injuries were concretely and indisputably disabling in a permanent way.

Often, the recognition of such permanent damages depend on a personal evaluation of the medical examiner, sometimes affected by an insufficient, or absent, or a specific clinical picture, and that is not necessarily derivable from the accident consequences. This type of approach can give cause for speculations, which can affect seriously the amount of the damages.

Normally when "micro-permanent" injuries are observed, the usual instruments (examination of the medical documentation provided by the damaged persons, subjectivity of the damaged person's statements and clinical objectivity) are insufficient for distinguishing those cases where a legitimate recognition can be made, in which a minimum indemnifiable impairment can be acknowledged, from those that instead are to be considered as speculations.

The presently adopted approach is also affected by lack of homogeneity of the data exchanged between the medical examiner that works on behalf of an insurance company and the insurance company same and for the absence of unified criteria in the exchange of such data.

Such data, furthermore, must be put in a national database, so called "accidents register", and, without a method that assists this step, relevant input and data treatment costs arise.

Data management editable software programs are presently available which may be downloaded from specific websites that are executed on special server, fed with data and sent back to the server in an encrypted format. However, such data managing is static and type of operating the data a type static and makes it difficult and slow to modify and to check the introduced data.

Therefore, it is necessary to reorganize the approach to the medical examinations, partly improving the methodology of analysis, partly providing instruments that are more precise with respect to those now available.

The above problems are in particular felt for micro-permanent impairments, which are largely the most common cases treated.

Of course, and even more so, the same methodology is used for all medical examinations of both public liability insurances and accident insurances, for all the levels up to 100% of disablement.

In EP1610253 a method is described for data collection and transmission comprising the steps of filling a predetermined number of fields of an interface file, or a visualization file, by an off-line data filling in process which relates to a medical examination. Afterwards, a step is provided of creating a structured file that contains the above mentioned data, and a step of transmitting the structured file to an insurance company.

However, the visualization file is split into different worksheets, which are dedicated to respective particular thematic sections. More in detail, the visualization file comprises a worksheet for introducing the data by a medical examiner who is in charge of the examination, a worksheet, which is different from the above one, for introducing the patient's data, still another data sheet for introducing the data about the accident, and so on. Filling in the visualization file by a medical examiner is therefore a complex operation, since the medical examiner has to jump all the time from a worksheet into another one, which causes, therefore, a remarkable waste of time. Such a structure of the visualization file increases the risk that the user, for example a medical examiner charged of the examination, makes mistakes when feeding the data, and therefore creates a wrong, or in any case an unfinished final examination file.

Moreover, a model of examination file as described in EP1610253, which as above said teaches the creation of a visualization file that must be filled in "off-line" with data; if it were used also for filling in data "on-line", it would have further drawbacks by creating a large, difficult-to-handle file, which requires a lot of time to be uploaded and forwarded to a target server.

### Summary of the invention

It is an object of the present invention to provide a method for obtaining and transmitting data for legal medicine services, which allows an easier use and a shorter analysis with respect to the known methods.

Another object is to provide a method which allows to calculate automatically damages, avoiding that a medical examiner has to perform manually the calculation.

It is also an object of the invention to provide a method that allows a medical examiner to rationally and quickly detect the examination data and to send them to the insurance company.

A further object is to provide a method for obtaining data in a structured format, the in addition to calculating the damages, also feeds an accident database, for instance a central accident record office.

Another object of the present invention is to allow a person who is in charge of deciding how to manage the accident damages, to evaluate in real terms all useful elements for a correct evaluation of the circumstances that require to be taken into account when a claim for damages has to be put in.

A further object of the present invention is to improve the communication exchange between the medical examiner and the insurance investigator when dealing with the injured person's private matter, with particular reference to "data privacy" when the injured person's lawyer asks the insurance company a copy of the report which has been drafted by its own medical examiner.

These and other objects are accomplished through the method for on-line drafting a medical examination, comprising the steps of:
- access by a user to a visualization structured file through a web interface, said visualization file comprising a plurality of fields intended to receive data that concern the examination, said visualization file computed by an operation database, said operation database run in a server;
- filling in by the user, in particular by a medical examiner who is in charge of the medical examination, at least one part of said fields intended to receive data, thus obtaining a filled in visualization file;
- cryptographic transmission of said filled in visualization file to said server;
   the main feature of the method is that said visualization file has a structure such that said plurality of fields is displayed in one data sheet, said fields intended to receive data that are organized as a plurality of thematic sections.

The particular structure of the visualization file allows therefore to a medical examiner, who is in charge of the examination, in a quite simple way to fill in the fields that are intended to receive data, so that the possibility of making mistakes is lowered. Furthermore, once the visualization file has been filled in in, it has a small size and it may accordingly be quickly uploaded to a data collection and data control server.

In particular, the visualization file is written in a language that is selected from the group comprised of:
- JavaScript;
- Active Server Pages (ASP);
- HTML;
- AJAX;
   or a combination thereof.

Advantageously, the step of access to said visualization file comprises the steps of:
- controlling the visualization file according to specific code tables;
- extracting the visualization file from the operation database which is resident on the remote server;
- obtaining the visualization file at a local PC;
- decoding the visualization file.

Advantageously, the step of filling in the fields that are intended to receive data by the user comprises a step of automatic calculation of the damages to be paid to an injured person.

In particular, the automatic calculation of the damages provides computing at least one of the following indexes:
- slight biological damage;
- permanent biological damage;
- temporary biological damage;
- moral damage.

In particular, the automatic calculation of the damages begins with filling in the following data by the user:
- an evaluation data of the invalidity in terms of invalidity percentage;
- an evaluation data of the invalidity in terms of days of invalidity;
- the date of birth of the injured person.

Advantageously, the cryptographic transmission to said server of said filled in visualization file is carried out by means of standard protocol SSL, *Secure Socket Layer,* to assure a safe data transmission.

In particular, a step of computing the visualization file may be carried out by a first operation database, i.e. by an interface database, which is resident in a first server during the filling in step, and by a second database, i.e. a main database, which is resident in a second server, when said filling in step is over. In particular, the first and the second server are connected through a data network and communicate with each other by means of an SSL protocol.

More in detail, when a user is connected with the website to open/create an examination, a part of the data that are resident in the main database is loaded to the interface database with which the user communicates during the filling in step. Once the the examination has been filled in by the user, the filled in visualization file is sent through interface database to the main database, to which the insurance companies may access for retrieving the data of interest.

Preferably, the visualization file comprises:
- a first part that concerns the clinical, anamnestic and documentary data report, i.e. the so-called "sensitive data" collected by a medical examiner during his own verification, a copy of which may be handed over to the injured person;
- a second part that contains the true evaluation of the subject as made by a medical examiner, said second part reserved to the insurance company;
- a third part that comprises a computer which is adapted to carry out said automatic calculation of said damages, said calculation performed by means of predetermined formulas.

Advantageously, each section is managed by the operation database independently from the other sections. This way, a plurality of users are enabled to fill in at the same time several sections of the examination process, though preventing a same section to be opened by more than one user at a time. In particular, the thematic sections are completely independent from one another when the file is loaded and the saved.

In particular, the first part of the visualization file comprises the following sections:
- the injured person's personal data;
- physiological and pathological anamnesis;
- the dynamics of the accident;
- the clinical course;
- the present clinical state;
- objective examination of the documentation.

In particular, the second part of the examination, which remains confidential, may comprise the following sections:
- a medical examiner's personal data;
- a specialized advice required by the insurance trusty person;
- a legal medicine evaluation;
- attached documents, in particular photographic documentation;
- legal medicine diagnosis,
- injury policy;
- any observations.
   Finally, the third part comprises the following section:

- public liability insurance.

Advantageously, the visualization file comprises:
- fields that may be filled in at the user's will;
- fields that are provided with a pulldown menu, in which only an entry can be set which is selected from a predetermined set of codes;
- parts of paragraphs that can be modified by the medical examiner, both in their content and in their format, in which the medical examiner may write any useful note, or which the medical examiner may remove or may not fill in;
- unmodifiable parts, for instance the titles of the sections.

Advantageously, said visualization file, once filled in, may be sent to a printer, or may be e-mailed to a user.

### Brief description of the drawings

The invention will be made clearer with the following description of an exemplary embodiment thereof, exemplifying but not limitative, with reference to the attached drawings in which:
- Fig. 1 shows in a flowchart with the main steps of the method for on-line drafting a legal medicine examination, according to the invention;
- Fig. 2 shows diagrammatically a possible set of hardware elements which is adapted to carry out the method of Fig. 1;
- Fig. 3 shows an example of a portion of a frame of a visualization file, according to the invention;
- figures 4A-4D show the structure of the visualization file of Fig. 3;
- Fig. 5 shows a portion of a frame of the visualization file.

### Description of preferred exemplary embodiments

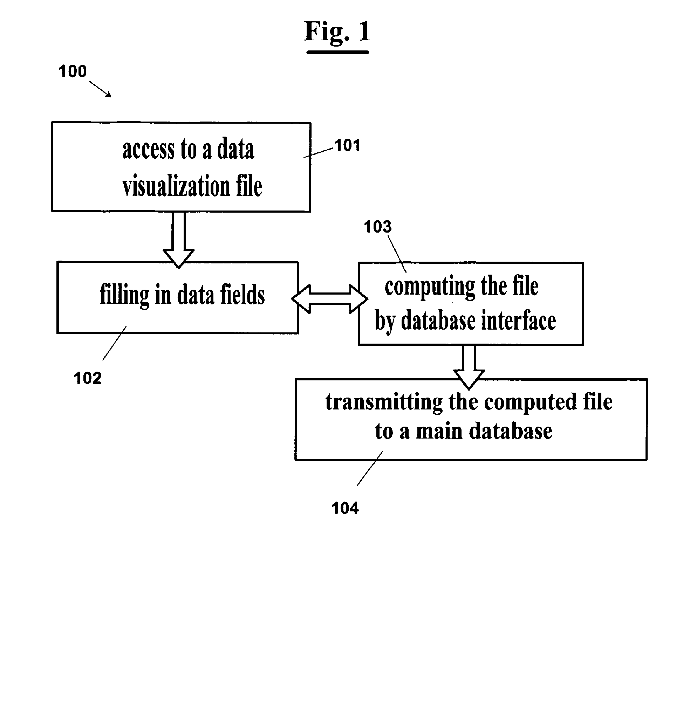

Fig. 1 shows in a flowchart 100 the main steps of the method for on-line drafting a medical examination, according to the invention.

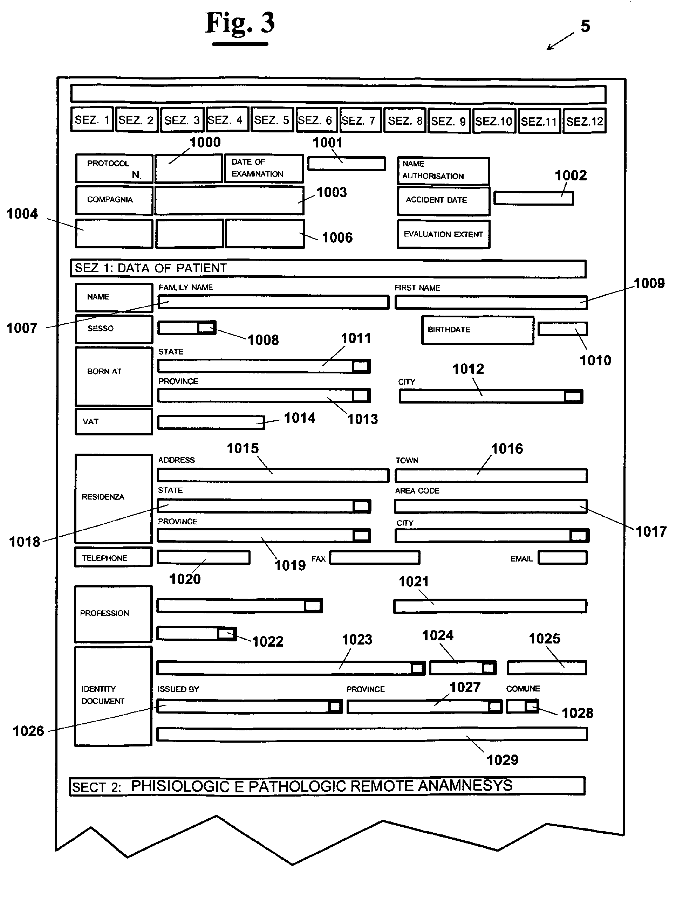

In particular, the method provides a first step in which a user, for example a medical examiner who is in charge of the examination, accesses through a web interface to a visualization file 5 which is executed in a server, box 101. Visualization file 5, as diagrammatically shown in Fig. 3, comprises a plurality of fields 1001-1030 that are intended to receive data that concern the examination, which can be displayed in one data sheet.

This technical feature allows the user to easily fill in fields intended to receive data of interest 1001-1030, preventing the medical examiner from making mistakes. Once the filling is step of the fields intended to receive data 1001-1030 has come to an end, a filled in visualization file is obtained, box 102.

While the medical examiner fills in visualization file 5, visualization file 5 is computed by an interface database that updates the filled in fields and checks formal correctness of the filling in process, box 103. These operations are assisted by the small size of file 5, which is due to the particular structure of the file.

The small size of the filled in visualization file 5 makes it possible to speed up its subsequent cryptographic transmission to the server, box 103, where the filled in visualization file can be displayed by the insurance companies, or by any other authorized users.

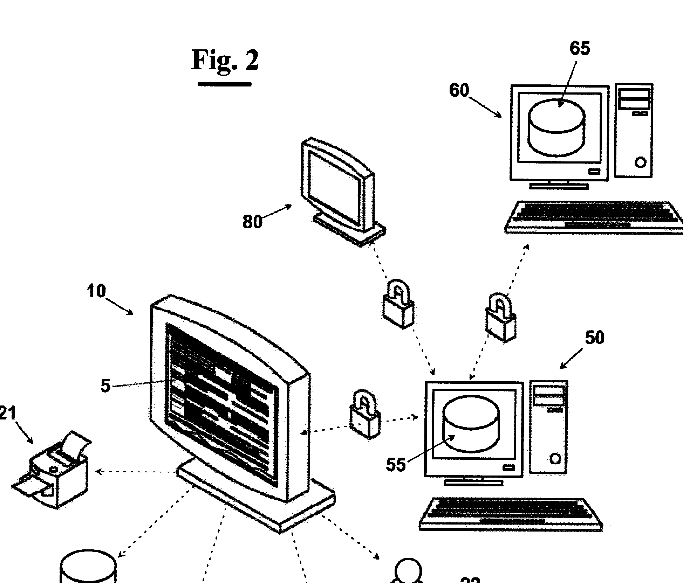

Fig. 2 diagrammatically shows the main hardware elements by which the method according to the invention is carried out, for obtaining and transmitting the medical examination data.

As described above, during the filling in step in visualization file 5 by the user, visualization file 5 is computed by an operation database 55, i.e. by an interface database which is resident in a server 50. In particular, visualization file 5 comprises a plurality of fields intended to receive data, a portion of which relates to the current examination as shown in Fig. 3.

Once a local PC 10 has been connected to server 50 through a data network, file 5 is opened and then displayed on PC 10 itself in a one-sheet format. This format comprises a prefixed number of thematic sections, for example 12 thematic sections, each comprising a prefixed number of fields intended to receive data of the above described plurality of fields, in order to allow the medical examiner who is in charge of the examination to fill them in. The peculiarity of the invention of displaying file 5 in a one-sheet format allows the medical examiner who is in charge of the examination to easily and quickly put the data in the proper respective fields according to a commonly used procedure.

In particular, in the example of Fig. 2 a first operation database, i.e. an interface database 55 is provided, which is resident in a first server 50 that computes file 5 during the filling in step; a second database, or main database 65, is also provided, which is resident in a second server 60, which computes file 5 once the filling in step has come to an end. Servers 50 and 60 are connected to each other through a data network, and communicate with each other by means of an SSL protocol. When a user is connected with the website for opening/creating a specific examination, a part of the data that are resident in main database 65 is loaded to interface database 55 with which a medical examiner communicates during the filling in step through PC 10. Once the step of filling in the examination frame has been ended by the user, the filled in visualization file is sent via interface database 55 to main database 65 to which the insurance companies can access to download the data of interest.

Once filling step has come to an end, file 5 may be sent to a printer 21, transformed in a .pdf file 24, or attached to an e-mail message 23, and sent to a user, or even stored in a such a specific national database 25, such as for Italy the "Casellario Centrale Infortuni - Banca Dati INAIL (INAIL)", the "Banca Dati Sinistri R.C. Auto (ISVAP)" and the like. A further possibility is to send filled in file 5 to a website, to allow other users to access it.

The technology used for filling and making the visualization file comprises the following languages: JavaScript, active Server Pages (ASP), HTML, AJAX.

The data transmitted between interface server 50, main server 60 and medical examiner's PC 10 are encrypted, for example, according to standard protocol SSL, *Secure Socket Layer,* in order to ensure a properly safe transmission of the data.

Visualization file 5 comprises a first part that concerns the clinical, anamnestic and documentary data report, i.e. the so-called "sensitive data" collected by a medical examiner during his verification, a copy of which may be handed over to the injured person. This part of file 5 comprises, in particular, a section for introducing the injured person's personal data, a section related to physiological and pathological anamnesis, a section where the data relative to the dynamics of the accident are introduced, a section related to the clinical course, a section relative to the injured person's present clinical state and a section related to the objective examination of the file.

File 5 comprises, moreover, a second part, which is reserved to the insurance company, and comprises the true evaluation of the subject, as provides by the medical examiner. In particular, this part of the file comprises a section that contains the medical examiner's personal data, a section comprising a specialized advice required by an insurance trusty person, a section comprising a legal medicine evaluation, a section where the documents are described and attached, in particular photographs of the accident, a section related to the legal medicine diagnosis, a section related to injury policy and a section for any remarks made by the medical examiner.

File 5 comprises, furthermore, a third part that comprises a computer which is adapted to automatically carry out the calculation of the damages to be paid to the injured person. In particular, the computer, starting from

The data transmitted between interface server 50, main server 60 and medical examiner's PC 10 are encrypted, for example, according to standard protocol SSL, *Secure Socket Layer,* in order to ensure a properly safe transmission of the data.

Visualization file 5 comprises a first part that concerns the clinical, anamnestic and documentary data report, i.e. the so-called "sensitive data" collected by a medical examiner during his verification, a copy of which may be handed over to the injured person. This part of file 5 comprises, in particular, a section for introducing the injured person's personal data, a section related to physiological and pathological anamnesis, a section where the data relative to the dynamics of the accident are introduced, a section related to the clinical course, a section relative to the injured person's present clinical state and a section related to the objective examination of the file.

File 5 comprises, moreover, a second part, which is reserved to the insurance company, and comprises the true evaluation of the subject, as provides by the medical examiner. In particular, this part of the file comprises a section that contains the medical examiner's personal data, a section comprising a specialized advice required by an insurance trusty person, a section comprising a legal medicine evaluation, a section where the documents are described and attached, in particular photographs of the accident, a section related to the legal medicine diagnosis, a section related to injury policy and a section for any remarks made by the medical examiner.

File 5 comprises, furthermore, a third part that comprises a computer which is adapted to automatically carry out the calculation of the damages to be paid to the injured person. In particular, the computer, starting from a predetermined set of data that have been introduced by a medical examiner, and using the formulas according to the law, for instance for Italy Law 57/2001 as presently modified, can quickly calculate the slight biological damage, permanent biological damage, temporary biological damage, moral damage, which are displayed in a specific section.

This way, a medical examiner can fill in the examination in a "traditional" way and give its evaluation in terms of invalidity percentage as days of invalidity. These data, together with other parameters as established by the above mentioned law, make it possible to calculate the sum to be proposed to settle the damages indemnity, which comprises the proposed medical approved by the medical examiner.

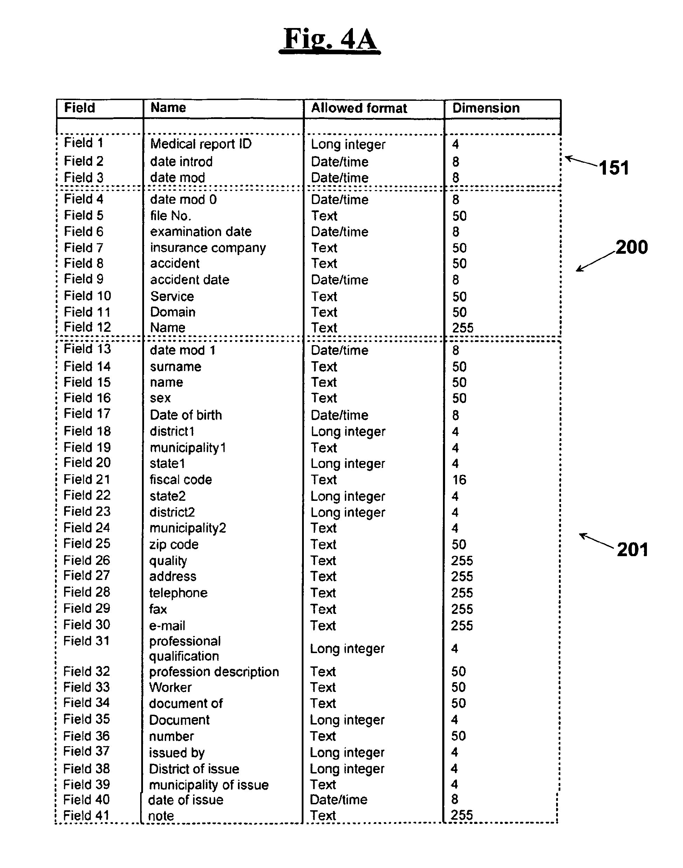

Each of the above described sections, which are displayed as highlighted before in one frame on PC 10 of a medical examiner who is in charge of filling the frame, comprises a set of structured fields, as shown in Figs. 4A to 4D.

For the sake of clearness, visualization file 5 is shown in a plurality of figures 4A to 4D, and relates to an example in which 157 fields are provided; for each field the name is displayed, as well as the format that must be used, e.g. "text", or "date/time", or a number format as "long integer", or "double precision number" i.e. a 64bit floating point format, or still a "memo" format, and the relative dimension.

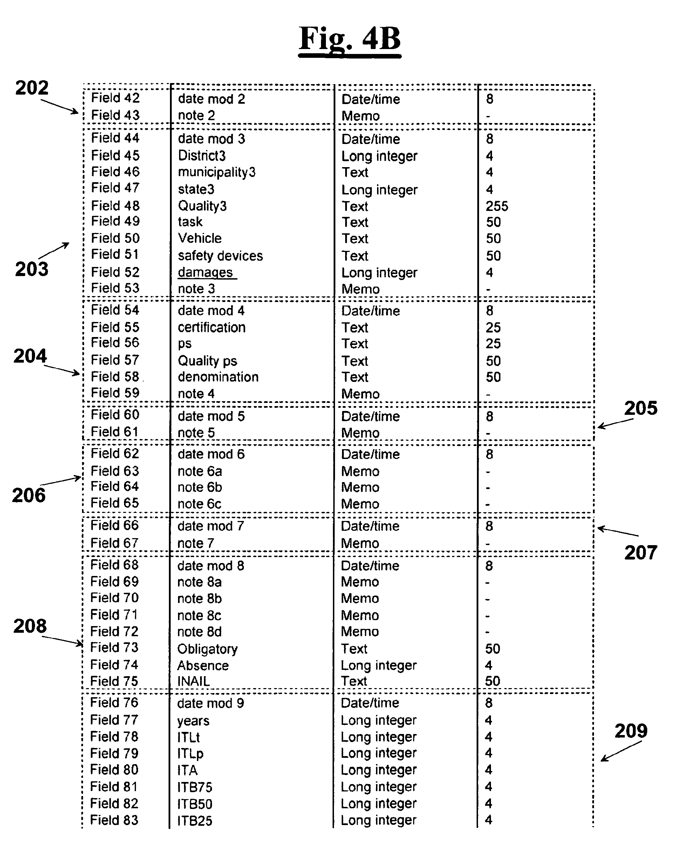

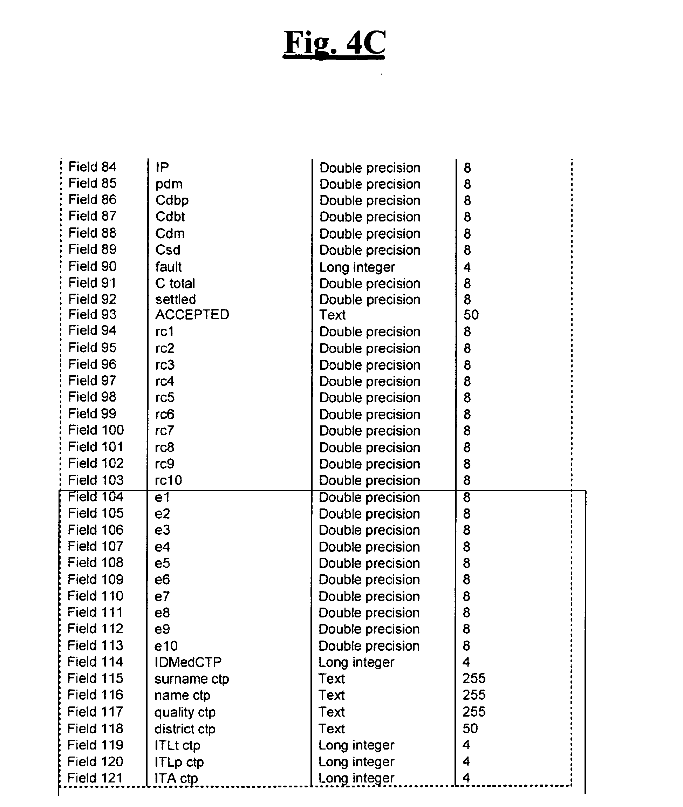

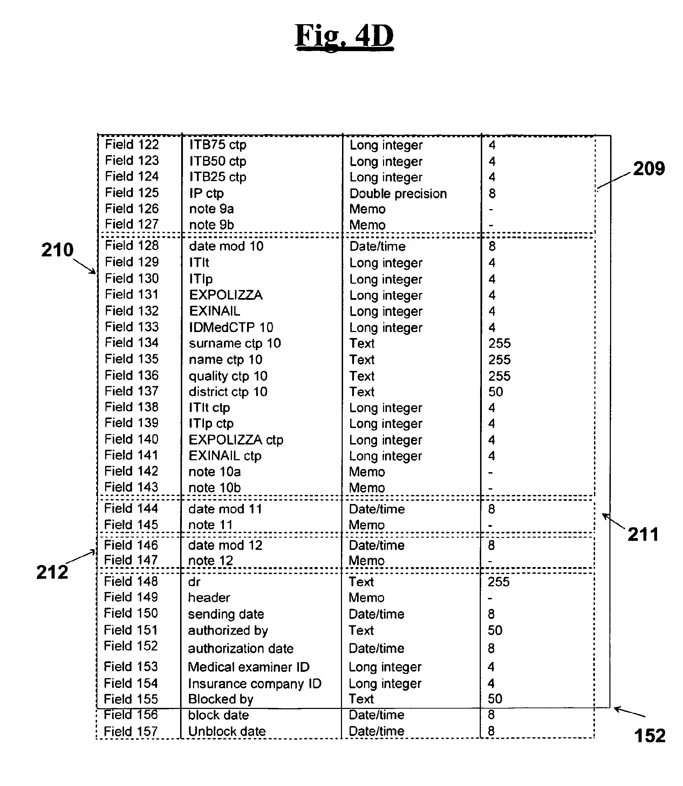

In Fig. 4, the fields from 1 to 157 that belong to a same section are bound by a dotted line frame. In particular, frames 201-212 represents twelve thematic sections; a header section 200 comprises fields 4 to 12 which refers to file identifying data, i.e. file number, field 5, examination date, field 6, insurance company, field 7. Moreover, the table of file 5, shown in Fig. 4, has two groups of control fields, namely fields 1 to 3 and fields 148 to 157 respectively bound by frames 151 and 152. The latter fields are used by the system to check whether the data have been introduced by the medical examiner using the proper format.

Sections 201 to 212 generally contain fields that may be filled in at the user's will, fields that are provided with a pulldown menu, in which only an entry can be set which is selected from a predetermined set of codes, parts of paragraph that can be modified by the medical examiner, both in their content and in their format, in which the medical examiner may write any useful note, or which the medical examiner may remove or may not fill in, unmodifiable parts, for example the titles of the sections.

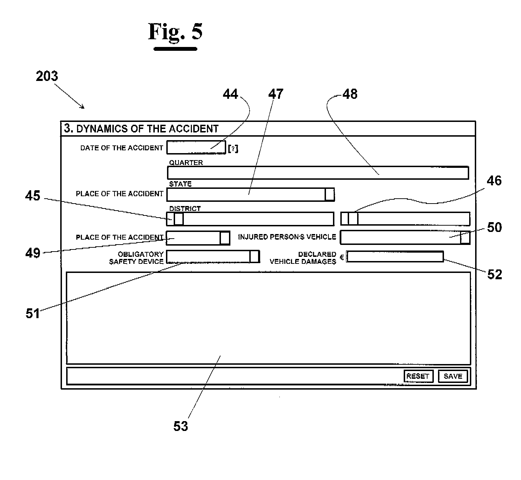

For example, the fields of section 3, i.e. "the dynamics of the accident", are enclosed for the sake of clearness in the dotted line frame 203. Moreover, a printout of section 3 itself is shown in Fig. 5 to allow immediate understanding of the correspondence between the structure of visualization file 5 and the file itself as displayed by the medical examiner's PC 10.

In particular, section 3 contains a field 44 for the accident date, which has therefore the "date/time" format. Furthermore, a plurality of data fields are provided which refers to the place where the accident has occurred, namely field 45 for the district, a field 46 for the municipality, a field 47 for the state and a field 48 for the quarter, all in "text", "long integer" format. Section three provides also a field 49 for describing the task the injured person has played in the accident, a field 50 for identifying the injured person's vehicle, a field 51 for describing the type of obligatory vehicle safety device, a field 52 which contains the declared vehicle damages in € and a field 53 for any useful note or remarks by the medical examiner who is in charge of the examination. In particular, the field 53, as other similar fields of the visualization file, can adapting itself to the content.

Each section is managed by the interface database independently from the other sections. This way, a plurality of users are enabled to fill in at the same time several sections of the examination file, however, preventing a same section to be opened by more than one user at a time

While the medical examiner carries out the step of filling in the visualization file, data are merged by merging respective tables and checking their integrity, coherence and quality.

The foregoing description of specific embodiments will so fully reveal the invention according to the conceptual point of view, so that others, by applying current knowledge, will be able to modify and/or adapt for various applications such embodiments without further research and without parting from the invention, and it is therefore to be understood that such adaptations and modifications will have to be considered as equivalent to the specific embodiments. The means and the materials to realise the different functions described herein could have a different nature without, for this reason, departing from the field of the invention. It is to be understood that the phraseology or terminology employed herein is for the purpose of description and not of limitation.

## Claims

1. A method for on-line drafting a medical examination, comprising the steps of:
- access by a user to a visualization structured file through a web interface, said visualization file comprising a plurality of fields intended to receive data that concern the examination, said visualization file computed by an operation database, said operation database executed in a server;
- filling in by said user at least one part of said fields intended to receive data, obtaining a filled in visualization file;
- cryptographic transmission of said filled in visualization file to said server;
**characterised in that** said visualization file has a structure such that said plurality of fields intended to receive data is displayed in one data sheet, said fields intended to receive data that are organized as a plurality of thematic sections.

2. A method, according to claim 1, wherein said visualization file is written in a language that is selected from the group comprised of:
- JavaScript;
- Active Server Pages (ASP);
- HTML;
- AJAX;
or a combination thereof.

3. A method, according to claim 1, wherein said step of access to said visualization file comprises the steps of:
- computing said visualization file according to specific code tables;
- extracting said visualization file from said operation database, which is resident on said remote server;
- acquisition of said visualization file at a local PC;
- decoding said visualization file.

4. A method, according to claim 1, wherein said step of filling said fields intended to receive data by said user comprises a step of automatic calculation of the damages to be paid to an injured person.

5. A method, according to claim 4, wherein said step of automatic calculation of the damages provides computing at least one of the following indexes:
- slight biological damage;
- permanent biological damage;
- temporary biological damage;
- moral damage.

6. A method, according to claim 4, wherein said step of automatic calculation of the damages begins with filling in the following data by the user:
- an evaluation data of the invalidity in terms of invalidity percentage;
- an evaluation data of the invalidity in terms of days of invalidity;
- the date of birth of the injured person.

7. A method, according to claim 1, wherein said cryptographic transmission to said server of said filled in visualization file is carried out by means of standard protocol SSL, Secure Socket Layer, to ensure the safety of the transmission the data.

8. A method, according to claim 1, wherein said computing of said visualization file is carried out by a first operation database, i.e. by an interface database, which is resident in a first server during said filling in step, and by a second database, i.e. a main database, which is resident in a second server, when said step of filling is over, wherein said first server and said second server are connected through a data network.

9. A method, according to claim 1, wherein said visualization file comprises:
- a first part that concerns the clinical, anamnestic and documentary data report, i.e. the so-called "sensitive data" collected by the user during his own verification, a copy of which may be handed over to the injured person;
- a second part that contains the true evaluation of the subject as made by a medical examiner, said second part reserved to the insurance company;
- a third part that comprises a computer which is adapted to carry out said automatic calculation of said damages, said calculation performed by means of predetermined formulas.

10. A method, according to claim 1, wherein each section is managed by said operation database independently from the other sections, such that a plurality of users are enabled to fill in at the same time several sections of the examination, however, preventing a same section to be opened by more than one user at a time.
